# EUROPÄISCHE PATENTSCHRIFT

(11) **EP 2 216 728 B1**
(45) Veröffentlichungstag und Bekanntmachung des Hinweises auf die Patenterteilung: **04.04.2012**
(21) Anmeldenummer: 09152035.3
(22) Anmeldetag: 04.02.2009
(51) Int. Cl.: G06F 19/00

(54) **Verfahren und Vorrichtung zur Bestimmung einer charakteristischen Eigenschaft einer anatomischen Struktur**
Method and device for determining a characteristic of an anatomical structure
Procédé et dispositif de détermination d'une propriété caractéristique d'une structure anatomique

(43) Veröffentlichungstag der Anmeldung: 11.08.2010
(73) Patentinhaber: BrainLAB AG, 85622 Feldkirchen (DE)
(72) Erfinder: Grünschläger, Frank, 80639 München (DE); Adamski, Martin, 81667, München (DE); Oschinski, Andreas, 80639 München (DE)
(74) Vertreter: Schwabe - Sandmair - Marx

(56) Entgegenhaltungen:
- US-A1- 2008 108 912
- CITAK ET AL: "Navigated femoral anteversion measurements: A new intraoperative technique" INJURY, JOHN WRIGHT AND SONS, BRISTOL, GB, Bd. 39, Nr. 4, 12. Februar 2008 (2008-02-12), Seiten 467-471, XP022527228 ISSN: 0020-1383
- YEON SOO LEE ET AL: "3D femoral neck anteversion measurements based on the posterior femoral plane in ORTHODOC(R) system" MEDICAL & BIOLOGICAL ENGINEERING & COMPUTING, SPRINGER, BERLIN, DE, Bd. 44, Nr. 10, 29. September 2006 (2006-09-29), Seiten 895-906, XP019439860 ISSN: 1741-0444
- PRASAD SIRIKONDA SIVA ET AL: "Femoral anteversion in infants: a method using ultrasound." SKELETAL RADIOLOGY AUG 2003, Bd. 32, Nr. 8, August 2003 (2003-08), Seiten 462-467, XP002534255 ISSN: 0364-2348
- MAHAISAVARIYA B ET AL: "MORPHOLOGICAL STUDY OF THE PROXIMAL FEMUR: A NEW METHOD OF GEOMETRICAL ASSESSMENT USING 3-DIMENSIONAL REVERSE ENGINEERING" MEDICAL ENGINEERING & PHYSICS, BUTTERWORTH-HEINEMANN, GB, Bd. 24, Nr. 9, 1. November 2002 (2002-11-01), Seiten 617-622, XP008069329 ISSN: 1350-4533
- SCHEYS L ET AL: "Personalized MR-based musculoskeletal models compared to rescaled generic models in the presence of increased femoral anteversion: Effect on hip moment arm lengths" GAIT & POSTURE, ELSEVIER, Bd. 28, Nr. 3, 1. Oktober 2008 (2008-10-01), Seiten 358-365, XP025677017 ISSN: 0966-6362 [gefunden am 2008-06-20]

## Beschreibung

Die vorliegende Erfindung betrifft ein Verfahren und eine Vorrichtung zur Bestimmung einer charakteristischen Eigenschaft einer anatomischen Struktur, insbesondere eines Knochens, aus der Lage von Abtastpunkten auf der Oberfläche der anatomischen Struktur.

Im Bereich der Medizin ist es oftmals notwendig, eine charakteristische Eigenschaft einer anatomischen Struktur zu kennen. Dabei handelt es sich insbesondere um eine geometrische Eigenschaft, beispielsweise die Lage einer Achse oder Ebene bzw. die Lage einer Achse oder Ebene relativ zu einer anderen Achse oder Ebene, wobei die anatomische Struktur beispielsweise ein Knochen ist.

Die geometrische Eigenschaft beschreibt insbesondere die relative Lage von Formrepräsentanten der anatomischen Struktur. Die Formrepräsentanten repräsentieren einen charakteristischen Aspekt der Form der anatomischen Struktur. Beispiele für Formrepräsentanten sind Geraden, Ebenen oder geometrische Figuren. Geometrische Figuren können eindimensional sein, wie z. B. Achsen oder Kreisbögen, zweidimensional sein, wie z. B. Vielecke und Kreise, oder dreidimensional sein, wie z. B. Quader, Zylinder und Kugeln. Formrepräsentanten können auch Teile einer anatomischen Struktur repräsentieren, beispielsweise Abschnitte eines Knochens. Die relative Lage zwischen den Formrepräsentanten kann in Bezugssystemen, z. B. durch Koordinaten oder Vektoren, beschrieben werden oder kann durch geometrische Größen, wie z. B. Länge, Winkel, Fläche, Volumen und Verhältnisse beschrieben werden. Die charakteristischen Aspekte, die durch die Formrepräsentanten repräsentiert werden, sind z. B. Symmetrieeigenschaften, die z. B. durch eine Symmetrieebene repräsentiert werden. Weiter ist ein charakteristischer Aspekt z. B. die Erstreckungsrichtung der anatomischen Struktur, die beispielsweise durch eine Längsachse repräsentiert wird. Weiter ist ein charakteristischer Aspekt z. B. die Querschnittsform einer anatomischen Struktur, die beispielsweise durch eine Ellipse dargestellt wird. Weiter ist ein charakteristischer Aspekt beispielsweise die Oberflächengestalt eines Teils der Struktur, die beispielsweise durch eine Ebene oder eine Halbkugel repräsentiert wird. Der charakteristische Aspekt stellt insbesondere eine Abstraktion der tatsächlichen Form oder eine Abstraktion einer Eigenschaft der tatsächlichen Form (wie z. B. die Symmetrieeigenschaft oder Längestreckung) dar.

Bei einem Oberschenkelknochen ist die charakteristische Eigenschaft beispielsweise der Winkel der Schenkelhalsachse bezogen auf die Achse des Schenkelschafts. Ist die anatomische Struktur eine Wirbelsäule oder ein Teil einer Wirbelsäule, so ist eine charakteristische Eigenschaft beispielsweise der Kyphose- oder Lordosewinkel zwischen zwei Wirbeln. Bei der charakteristischen Eigenschaft kann es sich auch um die Lage der anatomischen Struktur handeln, beispielsweise in Bezug auf eine andere anatomische Struktur.

Es sind verschiedene Methoden bekannt, eine charakteristische Eigenschaft aus der Lage von Abtastdaten, die Abtastpunkte auf der Oberfläche der anatomischen Struktur repräsentieren, zu bestimmen. Dazu wird beispielsweise ein Modell der anatomischen Struktur an die Abtastdaten angepasst und die Eigenschaft aus dem angepassten Modell abgeleitet, beispielsweise aus der relativen Lage von Landmarken des angepassten Modells zueinander. Eine andere Möglichkeit besteht darin, eine Achse bzw, eine Ebene als Ausgleichsachse bzw. Ausgleichsebene aus den Abtastdaten zu ermitteln und die charakteristische Eigenschaft daraus abzuleiten.

Der Beitrag "Morphological study of the proximal femur: A new method of geometrical Assessment using 3-dimensional reverse engineering" von Mahaisavariya B et al., veröffentlicht in Band 24, Nr. 9 von Medical Engineering & Physics, offenbart die Rekonstruktion eines Knochens aus geometrischen Grundformen wie Kreisen und Ellipsen basierend auf einer Menge von Abtastpunkten auf der Oberfläche des Knochens.

Es ist die Aufgabe der vorliegenden Erfindung, ein Verfahren und eine zugehörige Vorrichtung bereitzustellen, um die Bestimmung der charakteristischen Eigenschaft einer anatomischen Struktur mit erhöhter Genauigkeit zu ermöglichen.

Diese Aufgabe wird erfindungsgemäß dadurch gelöst, dass bei der Bestimmung der charakteristischen Eigenschaft der anatomischen Struktur ein Raumbereich definiert wird, bestimmt wird, welche der Abtastpunkte in dem Raumbereich liegen, und die als in dem Raumbereich liegend bestimmten Abtastpunkte zur Bestimmung der charakteristischen Eigenschaft ver-wendet werden. Bevorzugt werden überwiegend oder ausschließlich Abtastpunkte zur Bestimmung der charakteristischen Eigenschaft herangezogen, die in dem Raumbereich liegen. Werden überwiegend in dem Raumbereich liegende Abtastpunkte herangezogen, so bedeutet dies, dass beispielsweise mindestens 70 %, insbesondere mindestens 90 %, 95 % oder 99 % der Abtastpunkte in dem definierten Raumbereich liegen. Der definierte Raumbereich ist kleiner als die anatomische Struktur. Dies bedeutet, dass die anatomische Struktur nicht vollständig in dem definierten Raumbereich liegt. Der definierte Raumbereich umfasst bevorzugt nur einen Teil der Oberfläche der anatomischen Struktur.

Das erfindungsgemäße Verfahren ist insbesondere ein Datenverarbeitungsverfahren. Das Datenverarbeitungsverfahren wird bevorzugt durch technische Mittel, insbesondere einen Computer, durchgeführt. Der Computer umfasst insbesondere einen Prozessor und insbesondere einen Speicher, um insbesondere elektronisch die Daten zu verarbeiten. Insbesondere werden die beschriebenen Schritte des Berechnens von einem Computer ausgeführt. Schritte des Definierens von z. B. Bereichen oder Werten sind insbesondere Schritte des Festlegens von Daten im Rahmen des technischen Datenverarbeitungsverfahrens, insbesondere im Rahmen eines Programms. Schritte des Änderns stellen insbesondere eine Änderung der Daten mittels des Computers dar. Schritte des Ermittelns umfassen insbesondere die Abfrage von Werten, die an einer Schnittstelle des Computers anliegen und die durch technische Mittel, wie z. B. einer Abtasteinrichtung, erzeugt wurden. Diese Werte werden insbesondere von der Schnittstelle in Daten umgewandelt, die von dem Computer verarbeitet werden können.

Die oben genannten Abtastdaten werden dem erfindungsgemäßen Verfahren bevorzugt bereitgestellt. Werden Daten, Regionen, Bereiche oder Bilder "bereitgestellt", so bedeutet dies, dass sie für die Nutzung durch das erfindungsgemäße Verfahren bereit sind. Diesen Zustand des "Bereitstehens" können die Daten, Regionen, Bereiche oder Bilder z.B. durch Erfassen der Daten, Regionen, Bereiche oder Bilder (z.B. durch Analysegeräte) oder durch Eingeben der Daten, Regionen, Bereiche oder Bilder (z.B. über Schnittstellen) erreichen. Die Daten können diesen Zustand auch dadurch haben, dass sie in einem Speicher (z.B. ROM, CD, Festplatte) abgespeichert und so für eine Verwendung im Rahmen des erfindungsgemäßen Verfahrens bereit stehen.

Bei dem definierten Raumbereich handelt es sich beispielsweise um einen ebenflächig und/oder krummflächig begrenzten (geometrischen) Körper, z. B. einen Polyeder wie einen Quader oder einen Zylinder. Alternativ oder zusätzlich wird der Raumbereich durch mindestens eine Begrenzungsfläche, insbesondere eine Begrenzungsebene oder eine gekrümmte Begrenzungsfläche (z. B. Halbkugel), begrenzt. Beispielsweise wird der Raumbereich durch mindestens zwei Begrenzungsflächen, insbesondere zwei Begrenzungsebenen begrenzt, die bevorzugt zueinander parallel sind. Der definierte Raumbereich ist dann beispielsweise der Raumbereich zwischen den Begrenzungsflächen, insbesondere den Begrenzungsebenen, oder der Bereich auf der der anderen Begrenzungsfläche, insbesondere Begrenzungsebene, jeweils abgewandten Seite der Begrenzungsfläche, insbesondere Begrenzungsebene, also der Bereich außerhalb der Begrenzungsflächen bzw. Begrenzungsebenen.

Gemäß der Erfindung wird der definierte Raumbereich auf Basis von Landmarkendaten, die Landmarken der anatomischen Struktur repräsentieren, bestimmt, also insbesondere die Grenzen des Raumbereichs festgelegt. Bei einer Landmarke handelt es sich um einen definierten, charakteristischen Punkt einer anatomischen Struktur, der bei gleichartigen anatomischen Strukturen mehrerer Patienten stets identisch oder mit hoher Ähnlichkeit wiederkehrt. Typische Landmarken sind beispielsweise die Epikondylen eines Oberschenkelknochens oder die Spitzen der Querfortsätze bzw. des Domfortsatzes eines Wirbels.

Dabei wird ein Modell der anatomischen Struktur an die Landmarkendaten angepasst und der definierte Raumbereich aus dem angepassten Modell berechnet. Das Anpassen und/oder Berechnen erfolgt insbesondere automatisch mittels eines Prozessors. Bei dem Modell handelt es sich beispielsweise um ein generisches Modell der anatomischen Struktur, das aus einer Vielzahl von Punkten besteht. Diese Punkte definieren die Oberfläche des Modells der anatomischen Struktur. Die Punkte des Modells, insbesondere deren zwei- oder dreidimensionale Position, werden bevorzugt durch Modelldaten repräsentiert.

Bei der Anpassung des Modells an die Landmarkendaten, dem so genannten Matching oder Morphing, wird das generische Modell derart verändert, beispielsweise skaliert oder verzerrt, dass die Landmarken in den Landmarkendaten mit korrespondierenden Landmarken des Modells übereinstimmen. Dadurch wird das Modell in größtmögliche Übereinstimmung mit der tatsächlichen anatomischen Struktur gebracht. Dies erfolgt beispielsweise durch Verändern der Positionen der Punkte des Modells, also insbesondere durch Verändern der Modelldaten. Bevorzugt repräsentieren die Landmarkendaten die Lagen von genau drei Landmarken.

Die Ermittlung der Landmarkendaten, die dem Verfahren als Eingangsdaten bereitgestellt werden, kann direkt oder indirekt erfolgen. Bei einer direkten Ermittlung wird die entsprechende Landmarke unmittelbar abgetastet, bei einer indirekten Ermittlung werden zwei oder mehr Punkte abgetastet und die Landmarke wird aus diesen Punkten berechnet.

Optional wird nach der Berechnung der charakteristischen Eigenschaft der definierte Raumbereich verändert und die charakteristische Eigenschaft erneut berechnet. Dies ist insbesondere dann vorteilhaft, wenn sich die Plausibilität der charakteristischen Eigenschaft ermitteln lässt oder bei der Berechnung der charakteristischen Eigenschaft ein Maß für die Genauigkeit der Berechnung bestimmt wird.

In einer konkreten Anwendung handelt es sich bei der anatomischen Struktur um einen Oberschenkelknochen und bei der charakteristischen Eigenschaft um den Winkel der Schenkelhalsachse. Der Winkel der Schenkelhalsachse ist der Winkel zwischen dem Hals und dem Schaft des Oberschenkelknochens. Bei dieser Anwendung repräsentieren die Landmarkendaten beispielsweise einen Lateral-Mid-Neck-Point, den Mittelpunkt des Schenkelhalskopfes und den Mittelpunkt der Epikondylenachse des Oberschenkelknochens. Diese drei Punkte definieren eine Ebene, die als Koronarebene oder Femurebene bezeichnet wird. In dieser Ebene liegen sowohl die Halsachse als auch die Schaftachse des Oberschenkelknochens.

Der Lateral-Mid-Neck-Point ist ein Punkt in der Mitte des Schenkelhalses im lateralen Bereich, beispielsweise der Mittelpunkt des Bereiches, in dem der Piriformis am Oberschenkelknochen angreift. Zur Bestimmung des Mittelpunkts des Schenkelhalskopfes wird eine Vielzahl von Abtastwerten, beispielsweise 150, auf der Oberfläche des Schenkelhalskopfes vermessen und eine Ausgleichskugel durch die gemessenen Abtastpunkte gelegt, insbesondere mittels eines Prozessors. Der Mittelpunkt der Ausgleichskugel wird als Mittelpunkt des Schenkelhalskopfes festgelegt. Der Mittelpunkt der Epikondylenachse entspricht dem Mittelpunkt der Verbindungsgeraden zwischen dem lateralen und dem medialen Epikondylenpunkt. Die Erfindung betrifft weiterhin eine Vorrichtung zur Bestimmung einer charakteristischen Eigenschaft einer anatomischen Struktur mit einem Computer, der dazu eingerichtet ist, das vorstehend beschriebene Verfahren in Form der einzelnen Verfahrensschritte durchzuführen, sowie einer Abtasteinrichtung zur Ermittlung von Abtastpunkten auf der Oberfläche der anatomischen Struktur, um die Abtastdaten und/oder die Landmarkendaten zu erzeugen, die dem Computer zur Verarbeitung zugeführt werden. Bei der Abtasteinrichtung handelt es sich beispielsweise um einen Pointer, der mit einer Markervorrichtung, insbesondere einem Referenzstern, versehen ist. Über die Markervorrichtung bzw. den Referenzstern lässt sich die Lage, also die Position und die Ausrichtung, des Pointers und somit die Position der Spitze des Pointers bestimmen. Dazu wird die Markervorrichtung bzw. der Referenzstern beispielsweise mittels einer 3D-Kamera erfasst. Eine Markervorrichtung kann ein Referenzstern, ein Pointer und/oder ein einzelner oder mehrere Marker sein.

Aufgabe eines Markers ist, von einer Markererfassungseinrichtung (z.B. einer Kamera oder einem Ultraschallempfänger) erfasst zu werden, so dass seine Lage (d.h. Position und/oder Ausrichtung) im Raum ermittelt werden kann. Solche Marker können aktive Marker sein. Ein aktiver Marker emittiert beispielsweise elektromagnetische Strahlung bzw. Wellen, wobei diese Strahlung im infraroten, sichtbaren und/oder ultravioletten Spektralbereich liegen kann. Der Marker kann aber auch passiv sein, d.h. beispielsweise elektromagnetische Strahlung aus dem infraroten, sichtbaren und/oder ultravioletten Spektralbereich reflektieren. Dazu kann der Marker mit einer Oberfläche versehen sein, die entsprechende Reflektionseigenschaften besitzt. Es ist auch möglich, dass ein Marker elektromagnetische Strahlung bzw. Wellen reflektiert und/oder emittiert, die im Bereich der Radiofrequenzen oder bei Ultraschallwellenlängen liegt bzw. liegen. Ein Marker besitzt vorzugsweise sphärische Form bzw. eine kugelähnliche Gestalt und kann daher als Markerkugel bezeichnet werden, Marker können aber auch eine eckige, beispielsweise würfelige Gestalt aufweisen.

Ein Referenzstern bezeichnet eine Einrichtung, an der mehrere, vorteilhaft drei, Marker angebracht sind. Die Marker sind dabei ortsfest und vorteilhaft lösbar an dem Referenzstern angebracht, so dass eine bekannte (vorteilhaft feste) relative Lage der Marker zueinander entsteht. Die relative Lage der Marker zueinander kann für jeden im Rahmen eines chirurgischen Navigationsverfahrens verwendeten Referenzstern individuell unterschiedlich sein, um anhand der relativen Lage der Marker zueinander eine Identifikation des dazugehörigen Referenzsterns durch ein chirurgisches Navigationssystem zu ermöglichen. Damit können dann auch die Gegenstände (z. B. Instrumente und/oder Körperteile) identifiziert bzw. voneinander unterschieden werden, an welche der Referenzstern angebracht ist. Der Referenzstern dient dazu, in einem chirurgischen Navigationsverfahren eine Mehrzahl von Markern an einem Gegenstand (beispielsweise einem Knochen oder einem medizinischen Instrument) anzubringen, um die Lage des Gegenstandes im Raum (d.h. seine Position und/oder Ausrichtung) erfassen zu können. Ein solcher Referenzstern umfasst insbesondere eine Anbringmöglichkeit an dem Gegenstand (beispielsweise eine Schelle und/oder ein Gewinde) und/oder ein Halteelement, das für einen Abstand der Marker von dem Gegenstand sorgt (um insbesondere die Sichtbarkeit der Marker für eine Markererfassungseinrichtung zu unterstützen) und/oder mit dem Halteelement mechanisch verbundene Markerhalter, an die die Marker angebracht werden können. Wo es aus dem Zusammenhang klar wird, kann der Begriff Referenzstern auch einen Referenzstern mit wenigstens einem daran angebrachten Marker bezeichnen. Ein solches System aus einem Referenzstern und wenigstens einem Marker kann auch Markerstern genannt werden.

Die Erfindung betrifft weiterhin ein Computerprogramm, das, wenn es auf einem Computer ausgeführt wird oder geladen ist, den Computer dazu veranlasst, die Verfahrensschritte des vorstehend beschriebenen Verfahrens durchzuführen.

Im Rahmen der Erfindung können Computerprogrammelemente von Hardware und/oder Software (dies beinhaltet auch Firmware, im System abgespeicherte Software, Mikrocode usw.) verkörpert werden. Im Rahmen der Erfindung können Computerprogrammelemente die Form eines Computerprogrammprodukts annehmen, das durch ein auf einem Computer verwendbares oder computerlesbares Speicheimedium verkörpert werden kann, das auf einem Computer verwendbare oder computerlesbare Programmanweisungen, "Code" oder ein "Computerprogramm" umfasst, die bzw. der bzw. das auf dem genannten Medium zur Verwendung auf oder in Verbindung mit dem System, das die Anweisung ausführt, verkörpert sind bzw. ist. Ein solches System kann ein Computer sein, ein Computer kann eine Datenverarbeitungsvorrichtung mit Mitteln zum Ausführen der Computerprogrammelemente und/oder des erfindungsgemäßen Programms sein. Im Rahmen dieser Erfindung kann ein auf einem Computer verwendbares oder computerlesbares Medium irgendein Medium sein, dass das Programm zur Verwendung auf oder in Verbindung mit dem System, Apparat oder der Einrichtung, das bzw. der bzw. die die Anweisung ausführt, das Programm beinhalten, speichern, übermitteln, verbreiten oder transportieren kann. Das auf einem Computer verwendbare oder computerlesbare Medium kann z.B. ein elektronisches, magnetisches, optisches, elektromagnetisches, Infrarot- oder Halbleiter-System, ein solcher Apparat oder eine solche Einrichtung oder ein Ausbreitungsmedium wie z.B. das Internet sein, ist aber nicht auf diese Aufzählung beschränkt. Das auf einem Computer verwendbare oder computerlesbare Medium könnte sogar z.B. Papier oder ein anderes geeignetes Medium sein, auf das das Programm gedruckt ist, da das Programm elektronisch erfasst werden könnte durch z.B. optisches Scannen des Papiers oder anderen geeigneten Mediums und dann kompiliert, interpretiert oder andernfalls auf geeignete Weise verarbeitet werden könnte. Das Computerprogrammprodukt und jegliche Software und/oder Hardware, das bzw. die hier beschrieben werden, bilden in den beispielhaften Ausführungsformen die verschiedenen Mittel zum Ausführen der Funktionen der Erfindung. Insbesondere kann der Computer bzw. die Datenverarbeitungsvorrichtung eine Hinweisinformationsvorrichtung darstellen, die Mittel zum Ausgeben einer Hinweisinformation beinhaltet. Die Hinweisinformation kann visuell durch ein visuelles Anzeigemittel (z. B. einen Monitor und/oder eine Lampe) und/oder akustisch durch ein akustisches Anzeigemittel (z. B. einen Lautsprecher und/oder eine digitale Sprachausgabeeinrichtung) und/oder taktil durch ein taktiles Anzeigemittel (z. B. ein in ein Instrument eingebautes vibrierendes Element bzw. Vibrationselement) beispielsweise an einen Benutzer ausgegeben werden.

Es ist möglich, einzelne oder alle Merkmale der beschriebenen Ausgestaltungsformen miteinander zu kombinieren oder nicht erfindungswesentliche Merkmale aus Merkmalskombinationen wegzulassen.

Die vorliegende Erfindung soll anhand eines Ausführungsbeispiels näher erläutert werden. Dabei zeigt:
- Figur 1: eine schematische Darstellung eines Oberschenkelknochens,
- Figur 2: eine Modell-Schenkelhalsachse mit zwei Abtastpunkten,
- Figur 3: einen Teil des Oberschenkelknochens mit zwei Begrenzungsebenen,
- Figur 4: Abtastpunkte zur Bestimmung der Schenkelhalsachse, und
- Figur 5: eine Vorrichtung zur Bestimmung einer charakteristischen Eigenschaft einer anatomischen Struktur.

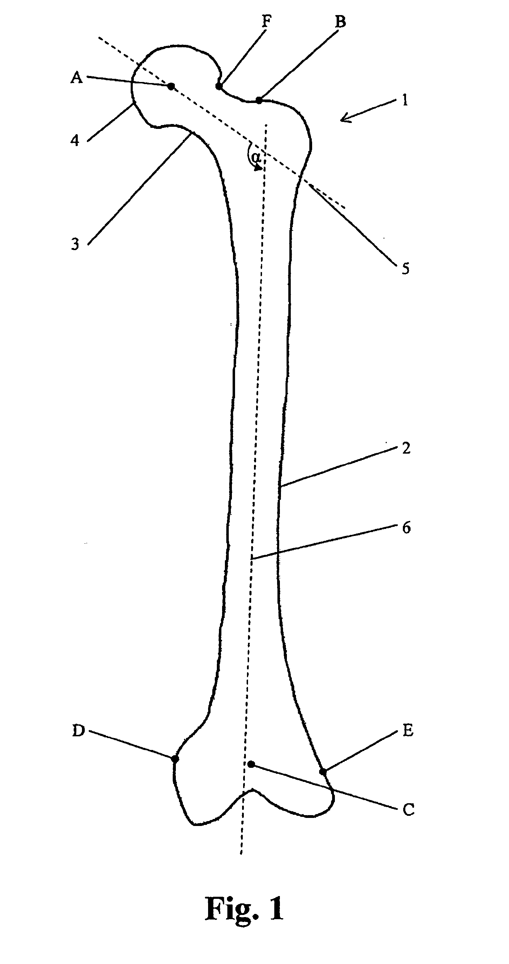

Die Figur 1 zeigt schematisch einen Oberschenkelknochen 1 mit einem Schaft 2, einem Hals 3 und einem Kopf 4. Eine charakteristische Eigenschaft des Knochens 1 ist der Schenkelhalswinkel α zwischen der Schenkelhalsachse 5 und der Schaftachse 6.

Zur Berechnung des Schenkelhalswinkels α werden zunächst Landmarkendaten erzeugt, indem drei Landmarken gemessen werden. Die erste Landmarke ist der Mittelpunkt A des Schenkelhalskopfes 4. Zur Bestimmung des Punktes A wird eine Vielzahl von Abtastpunkten auf dem Schenkelhalskopf aufgenommen. Die Anzahl aufgenommener Abtastpunkte liegt beispielsweise zwischen 100 und 200, insbesondere bei 150. Aus den aufgenommenen Abtastpunkten wird der Mittelpunkt A des Schenkelhalskopfes 4 berechnet, beispielsweise als Mittelpunkt einer Ausgleichskugel bezüglich der Abtastpunkte.

Die zweite Landmarke stellt der Lateral-Mid-Neck-Point B dar. Dies ist ein Punkt in der Mitte des Schenkelhalses 3 im lateralen Bereich, beispielsweise der Mittelpunkt der Fläche, in der der Piriformis am Oberschenkelknochen 1 angreift.

Die dritte Landmarke ist der Mittelpunkt C der Epikondylenachse. Der Mittelpunkt C der Epikondylenachse ist der Mittelpunkt der Verbindungsgeraden zwischen dem medialen Epikondylenpunkt D und dem lateralen Epikondylenpunkt E. Zur Berechnung des Mittelpunkts C werden die beiden Epikondylenpunkte D und E abgetastet und der Mittelpunkt zwischen ihnen berechnet

Die drei Landmarken A, B und C dienen als Grundlage zur Definition eines Raumbereichs. Ausschließlich Abtastpunkte, die in diesem definierten Raumbereich liegen, werden in einem späteren Verfahrensschritt bei der Bestimmung des Winkels α der Schenkelhalsachse 5 berücksichtigt. Alternativ werden überwiegend Abtastpunkte berücksichtigt, die in diesem definierten Raumbereich liegen. Dies bedeutet, dass beispielsweise mindestens 70 %, mindestens 90 %, mindestens 95 % oder mindestens 99 % der berücksichtigten Abtastpunkte innerhalb des definierten Raumbereichs liegen. Diese Alternative betrifft auch alle weiteren Ausführungen.

Zur Definition dieses Raumbereichs wird zunächst aus den Landmarkendaten die Koronarebene, die auch als Femurebene bezeichnet wird, des Oberschenkelknochens 1 bestimmt. Die Koronarebene ist diejenige Ebene, in der die Landmarken A, B und C liegen. Anschließend wird ein generisches Modell eines Oberschenkelknochens derart angepasst, dass die von den Landmarkendaten repräsentierten Landmarken A, B und C mit den Landmarken des Modells korrespondieren. Dazu wird das Modell so ausgerichtet, dass die Schenkelhalsachse und die Schaftachse des Modells in der Koronarebene liegen. Anschließend wird das Modell so skaliert und/oder verzerrt, dass der Mittelpunkt A des Schenkelhalskopfes und der Mittelpunkt C der Epikondylenachse mit den korrespondierenden Punkten des Modells übereinstimmen. Aussichten, skalieren oder verzerren der Modells bedeutet eine Veränderung der Modelldaten.

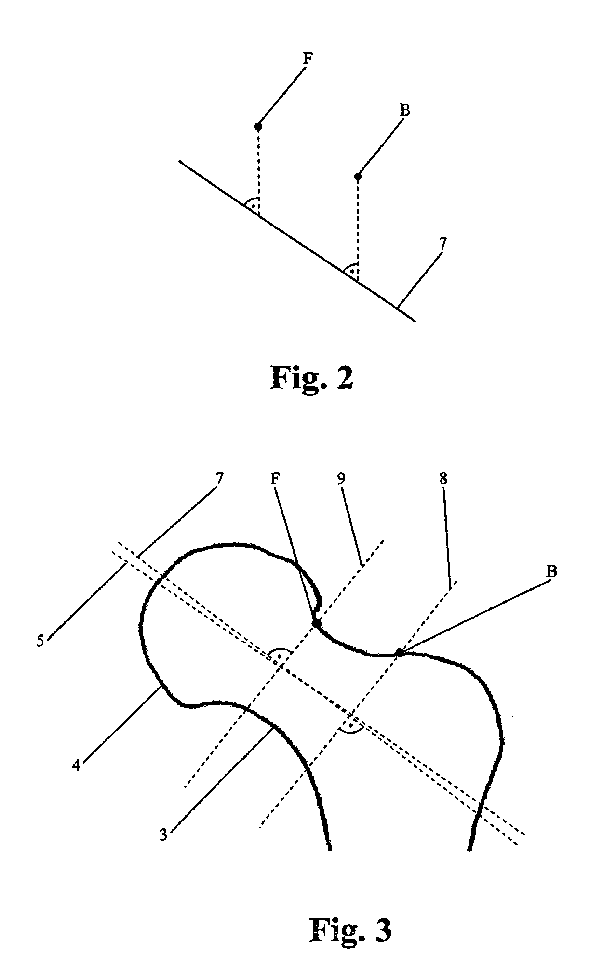

Da das angepasste Modell nicht notwendigerweise vollständig der Form des tatsächlichen Oberschenkelknochens 1 entspricht, entspricht der Winkel der Modell-Schenkelhalsachse üblicherweise nicht exakt dem tatsächlichen Winkel α der Schenkelhalsachse 5. Die Modell-Schenkelhalsachse, die in den Figuren 2 und 3 mit dem Bezugszeichen 7 versehen ist, dient jedoch als Ausgangspunkt zur Definition des Raumbereichs. Bei der Modell-Schenkelhalsachse handelt es sich um die Schenkelhalsachse 7 des generischen Modells.

Der definierte Raumbereich wird im vorliegenden Ausführungsbeispiel durch zwei parallele Ebenen beschränkt, auf denen die Modell-Schenkelhalsachse 7 jeweils senkrecht steht. Bei der Berechnung des Winkels α der Schenkelhalsachse 5 werden nur diejenigen Abtastpunkte der Oberfläche des Oberschenkelknochens 1 berücksichtigt, die zwischen diesen beiden parallelen Ebenen 8 und 9 liegen. Die Ebenen werden insbesondere durch Ebenendaten repräsentiert, die bevorzugt einen Punkt in der Ebene und einen Normalenvektor auf der Ebene umfassen.

Die Bestimmung der beiden Begrenzungsebenen 8 und 9 ist in Figur 2 dargestellt. Die erste Begrenzungsebene 8 ist diejenige Begrenzungsebene, auf der die Modell-Schenkelhalsachse 7 senkrecht steht und in der der Lateral-Mid-Neck-Point B liegt. Zur Bestimmung der zweiten Begrenzungsebene 9 wird ein Head-Neck-Junction-Point F herangezogen, der den Übergang vom Schenkelhals 3 in den Schenkelkopf 4 markiert und beispielsweise ebenfalls abgetastet wird. Die Begrenzungsebene 9 wird so gelegt, dass sie den Punkt F enthält.

Optional werden die Begrenzungsebenen 8 und 9 vor der Berechnung der charakteristischen Eigenschaft der anatomischen Struktur, also der Berechnung des Winkels der Schenkelhalsachse, entlang ihrer Flächennormalen gegeneinander verschoben, so dass sich ihr Abstand verändert. Der ursprüngliche Abstand wird beispielsweise um eine absolute Distanz, z. B. 1 mm, 2 mm, 5 mm oder 1 cm, erhöht oder verringert oder mit einem Faktor, beispielsweise 0,75, 0,8, 0,9, 1,1, 1,2 oder 1,25, multipliziert. Bevorzugt werden beide Begrenzungsebenen gegenüber ihrer ursprünglichen Position um die gleiche Distanz verschoben.

Die Figur 3 zeigt einen Ausschnitt des Oberschenkelknochens 1 mit der tatsächlichen Schenkelhalsachse 5, der Modell-Schenkelhalsachse 7 sowie den beiden Begrenzungsebenen 8 und 9. Die Begrenzungsebenen 8 und 9 bilden jeweils einen rechten Winkel mit der Modell-Schenkelhalsachse 7. Abtastpunkte der Oberfläche des Oberschenkelknochens 1, die nicht zwischen den beiden Begrenzungsebenen 8 und 9 liegen, werden bei der Berechnung des Winkels α der Schenkelhalsachse 5 nicht berücksichtigt.

Optional kann der definierte Raumbereich weiter beschränkt werden, beispielsweise auf einen quaderförmigen oder zylinderförmigen Bereich. Bei einem quaderförmigen Bereich liegen zwei gegenüberliegende Quaderflächen in den Begrenzungsebenen 8 und 9. Ein zweites Paar gegenüberliegender Quaderflächen liegt beispielsweise parallel zu der Koronarebene, das dritte Paar gegenüberliegender Quaderflächen steht beispielsweise senkrecht auf der Koronarebene. Der Abstand der das zweite bzw. das dritte Paar bildenden Quaderflächen wird bevorzugt jeweils zwischen 4 cm und 7 cm festgelegt oder in Abhängigkeit vom Abstand der Begrenzungsebenen 8 und 9 gewählt, beispielsweise als 0,5, 0,75, 1, 1,25 oder 1,5 mal dieser Abstand.

Bei einem zylinderförmigen Raumbereich liegen die Stirnflächen des Zylinders in den Begrenzungsebenen 8 und 9, die Zylinderachse entspricht der Modell-Schenkelhalsachse 7. Der Radius des Zylinders ist entweder ein absoluter Wert, beispielsweise 2 cm, 3 cm oder 4 cm, oder ein relativer Wert, beispielsweise 0,1, 0,2, 0,25, 0,5, 1, 1,25, 1,5 oder 2 mal der Abstand zwischen den beiden Begrenzungsebenen 8 und 9.

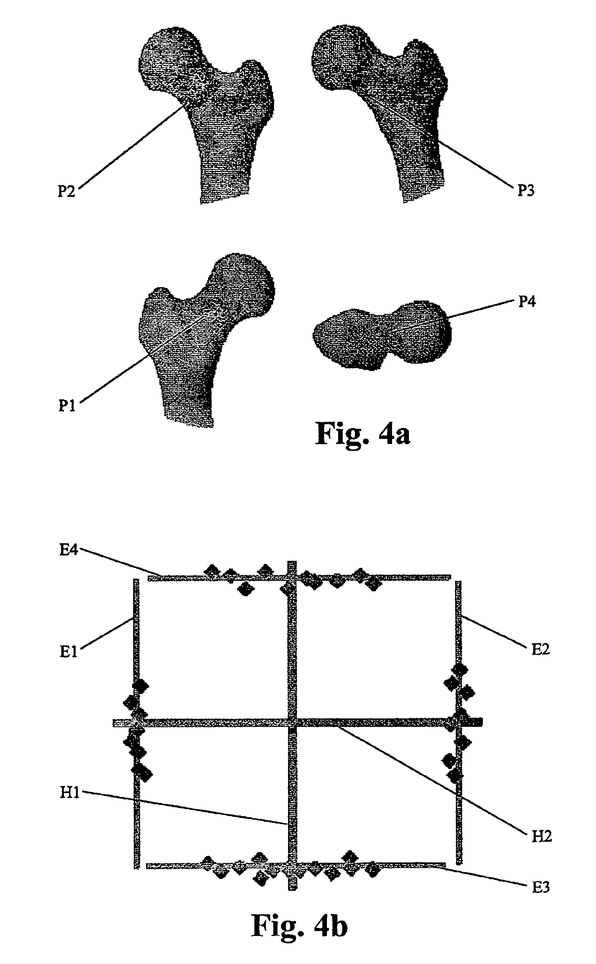

Die Bestimmung der Lage der Schenkelhalsachse 5 wird anhand der Figur 4 erläutert. Die Figur 4a zeigt vier Mengen von Abtastpunkten an der posterioren, superioren, anterioren und inferioren Seite des Schenkelhalses 3. Diese vier Punktmengen sind in der Figur 4a mit P1, P2, P3 und P4 bezeichnet. Sie liegen jeweils in dem Raumbereich, der wie vorstehend beschrieben definiert bzw. begrenzt wurde.

Die Figur 4b zeigt die vier Punktmengen P1, P2, P3 und P4 aus einer seitlichen Ansicht, die exakt oder ungefähr in Richtung der Schenkelhalsachse verläuft. Durch jede der Punktmengen wird eine Ausgleichsebene gelegt, beispielsweise derart, dass die Summe der Abstände oder die Summe der quadrierten Abstände aller Abtastpunkte in der jeweiligen Punktmenge zu der Ausgleichsebene minimiert wird. Die Ausgleichsebenen sind mit den Bezugszeichen E1, E2, E3 und E4 versehen, wobei die Ausgleichsebene E1 zur Punktmenge P1 gehört und so weiter.

In einem weiteren Schritt werden zwei Hilfsebenen H1 und H2 berechnet. Die Hilfsebene H1 liegt mittig zwischen der posterioren Ausgleichsebene E1 und der anterioren Ausgleichsebene E2, die Hilfsebene H2 liegt mittig zwischen der inferioren Ausgleichsebene E3 und der superioren Ausgleichsebene E4. Eine mittige Lage zwischen zwei Ebenen bedeutet bei parallelen Ebenen, dass die Hilfsebene parallel zu den beiden Ebenen liegt und zu beiden Ebenen den gleichen Abstand aufweist, und bei nicht parallelen Ebenen, dass die Hilfsebene die Winkelhalbierende zwischen den beiden Ebenen darstellt. Die Schenkelhalsachse 5 ist nun die Schnittgerade zwischen der ersten Hilfsebene H1 und der zweiten Hilfsebene H2.

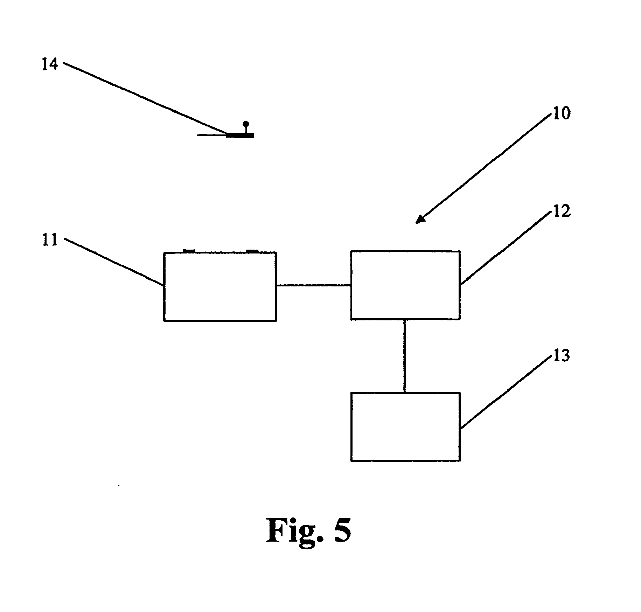

Die Figur 5 zeigt schematisch eine Vorrichtung 10 zur Bestimmung einer charakteristischen Eigenschaft einer anatomischen Struktur. Die Vorrichtung 10 weist eine 3D-Kamera 11, einen Computer 12 und eine Anzeigevorrichtung 13 auf. Die 3D-Kamera 11 ist dazu ausgebildet, die Lage, also die Position und die räumliche Ausrichtung, eines Pointers 14 zu ermitteln. Der Pointer 14 weist eine Spitze auf, die mit einem abzutastenden Punkt in Kontakt gebracht werden kann. Weiterhin weist der Pointer 14 eine nur symbolisch dargestellte Markervorrichtung in Form eines Referenzstems auf.

Die 3D-Kamera 11 weist zwei in der Figur 5 nur angedeutete Objektive auf, mittels derer ein stereoskopisches Bild des Pointers 14 und der daran befindlichen Markervorrichtung aufgenommen wird. Aus dem stereoskopischen Bild und der Position der 3D-Kamera 11 lässt sich die Lage des Pointers 14, insbesondere von dessen Spitze, berechnen, beispielsweise in der 3D-Kamera 11 oder dem mit der 3D-Kamera 11 verbundenen Computer 12.

Der Computer 12 ist dazu eingerichtet und darauf programmiert, die Verfahrensschritte zur Bestimmung einer charakteristischen Eigenschaft einer anatomischen Struktur auszuführen. Dazu sind Informationen wie die Beschaffenheit des Pointers 14 oder des generischen Modells der anatomischen Struktur hinterlegt.

Mit dem Computer 12 verbunden ist eine Anzeigevorrichtung 13. Über die Anzeigevorrichtung 13 und eine nicht dargestellte Eingabeeinrichtung kann der Computer 12 mit einem Bediener der Vorrichtung 10 kommunizieren. Auf der Anzeigevorrichtung 13 wird beispielsweise die nächste abzutastende Landmarke, beispielsweise hervorgehoben in einer Abbildung des generischen Modells der anatomischen Struktur, dargestellt. Alternativ oder zusätzlich wird auf der Anzeigevorrichtung 13 die charakteristische Eigenschaft der anatomischen Struktur und/oder eine Hinweisinformation dargestellt. Eine mögliche Hinweisinformation weist darauf hin, dass sich der Punkt, an dem sich die Spitze des Pointers 14 befindet, außerhalb des definierten Raumbereichs befindet. Eine weitere Möglichkeit für die Hinweisinformation ist, dass genügend Messdaten zur Bestimmung der charakteristischen Eigenschaft der anatomischen Struktur aufgenommen wurden.

## Patentansprüche

1. Computerimplementiertes Verfahren zur Bestimmung einer charakteristischen Eigenschaft einer anatomischen Struktur (1), insbesondere eines Knochens, aus Abtastdaten, die die Lage von Abtastpunkten auf der Oberfläche der anatomischen Struktur (1) repräsentieren, **dadurch gekennzeichnet, dass** ein Raumbereich definiert wird, wobei die anatomische Struktur nicht vollständig in dem definierten Raumbereich liegt, bestimmt wird, welche der Abtastpunkte in dem Raumbereich liegen, und die als in dem Raumbereich liegend bestimmten Abtastpunkte zur Bestimmung der charakteristischen Eigenschaft verwendet werden, wobei der definierte Raumbereich aus Landmarkendaten bestimmt wird, die Landmarken (A, B, C) der anatomischen Struktur (1) repräsentieren, indem ein Modell der anatomischen Struktur (1) an die Landmarkendaten angepasst wird und der definierte Raumbereich aus dem angepassten Modell berechnet wird.

2. Verfahren nach Anspruch 1, **dadurch gekennzeichnet, dass** es sich bei dem definierten Raumbereich um einen ebenflächig und/oder krummflächig begrenzten (geometrischen) Körper, z. B. einen Polyeder wie einen Quader oder einen Zylinder handelt.

3. Verfahren nach Anspruch 1 oder 2, **dadurch gekennzeichnet, dass** der Raumbereich durch mindestens eine Begrenzungsebene (8, 9) definiert wird.

4. Verfahren nach Anspruch 1, **dadurch gekennzeichnet, dass** die Landmarkendaten die Lage von drei Landmarken (A, B, C) repräsentieren.

5. Verfahren nach einem der Ansprüche 1 bis 4, **dadurch gekennzeichnet, dass** nach der Berechnung der charakteristischen Eigenschaft der definierte Raumbereich verändert und die charakteristische Eigenschaft erneut berechnet wird.

6. Verfahren nach einem der Ansprüche 1 bis 5, **dadurch gekennzeichnet, dass** es sich bei der anatomischen Knochenstruktur (1) um einen Oberschenkelknochen und bei der charakteristischen Eigenschaft um den Winkel (α) der Schenkelhalsachse (5) handelt.

7. Verfahren nach Anspruch 6, **dadurch gekennzeichnet, dass** es sich bei den Landmarken, die von den Landmarkendaten repräsentiert werden, um einen Lateral Mid-Neck Point (B), den Mittelpunkt (A) des Schenkelhalskopfs (4) und den Mittelpunkt (C) der Epikondylenachse des Oberschenkelknochens (1) handelt.

8. Vorrichtung zur Bestimmung einer charakteristischen Eigenschaft einer anatomischen Struktur (1), insbesondere eines Knochens, mit einem Computer, der dazu eingerichtet ist, das Verfahren nach einem der Ansprüche 1 bis 7 durchzuführen, sowie einer Abtasteinrichtung zur Ermittlung von Abtastpunkten auf der Oberfläche der anatomischen Struktur (1), um die Abtastdaten und/oder die Landmarkendaten zu erzeugen, die dem Computer zur Verarbeitung zugeführt werden.

9. Computerprogramm, das, wenn es auf einem Computer ausgeführt wird oder geladen ist, den Computer dazu veranlasst, die Verfahrensschritte eines Verfahrens nach einem der Ansprüche 1 bis 7 durchzuführen.

## Claims

1. A computer-implemented method for determining a characteristic property of an anatomical structure (1), in particular a bone, from scan data which represent the position of scan points on the surface of the anatomical structure (1), **characterised in that** a spatial region is defined, wherein the anatomical structure does not completely lie in the defined spatial region, the scan points which lie in the spatial region are determined, and the scan points which are determined to be lying in the spatial region are used to determine the characteristic property, wherein the defined spatial region is determined from landmark data which represent landmarks (A, B, C) of the anatomical structure (1), by adapting a model of the anatomical structure (1) to the landmark data and calculating the defined spatial region from the adapted model.

2. The method according to claim 1, **characterised in that** the defined spatial region is a (geometric) body which is delimited over a planar area and/or a curved area, for example a polyhedron such as a cuboid or a cylinder.

3. The method according to claim 1 or 2, **characterised in that** the spatial region is defined by at least one delimiting plane (8, 9).

4. The method according to claim 1, **characterised in that** the landmark data represent the position of three landmarks (A, B, C).

5. The method according to any one of claims 1 to 4, **characterised in that** after the characteristic property has been calculated, the defined spatial region is altered and the characteristic property is recalculated.

6. The method according to any one of claims 1 to 5, **characterised in that** the anatomical bone structure (1) is a femoral bone, and the characteristic property is the angle (α) of the femoral neck axis (5).

7. The method according to claim 6, **characterised in that** the landmarks represented by the landmark data are a lateral mid-neck point (B), the mid-point (A) of the head (4) of the femoral neck and the mid-point (C) of the epicondylar axis of the femoral bone (1).

8. A device for determining a characteristic property of an anatomical structure (1), in particular a bone, comprising: a computer which is designed to perform the method according to any one of claims 1 to 7; and a scanning device for ascertaining scan points on the surface of the anatomical structure (1), in order to generate the scan data and/or landmark data which are supplied to the computer for processing.

9. A computer program which, when it is executed or loaded on a computer, causes the computer to perform the method steps of a method according to any one of claims 1 to 7.

## Revendications

1. Procédé mis en oeuvre par ordinateur pour déterminer une propriété caractéristique d'une structure anatomique (1), en particulier d'un os, à partir de données de balayage qui représentent la position de points de balayage sur la surface de la structure anatomique (1), **caractérisé en ce qu'**il comporte les étapes consistant à définir une région spatiale, la structure anatomique n'étant pas entièrement située dans la région spatiale définie, déterminer les points de balayage qui se situent dans la région spatiale et utiliser les points de balayage déterminés comme étant situés dans la région spatiale afin de déterminer la propriété caractéristique, dans lequel la région spatiale définie est déterminée à partir de données de points de repère qui représentent des points de repère (A, B, C) de la structure anatomique (1), en adaptant un modèle de la structure anatomique (1) aux données de points de repère et en calculant la région spatiale définie à partir du modèle adapté.

2. Procédé selon la revendication 1, **caractérisé en ce que** la région spatiale définie est un corps (géométrique) délimité par une surface plane et/ou courbe, par exemple un polyèdre tel qu'un parallélépipède ou un cylindre.

3. Procédé selon la revendication 1 ou 2, **caractérisé en ce que** la région spatiale est définie par au moins un plan de délimitation (8, 9).

4. Procédé selon la revendication 1, **caractérisé en ce que** les données de points de repère représentent la position de trois points de repère (A, B, C).

5. Procédé selon l'une quelconque des revendications 1 à 4, **caractérisé en ce que** la région spatiale définie est changée après le calcul de la propriété caractéristique et la propriété caractéristique est recalculée.

6. Procédé selon l'une quelconque des revendications 1 à 5, **caractérisé en ce que** la structure anatomique osseuse (1) est un os fémoral et la propriété caractéristique est l'angle (α) de l'axe de col fémoral (5).

7. Procédé selon la revendication 6, **caractérisé en ce que** les points de repère représentés par les données de points de repère sont un point milieu de col latéral (B), le point milieu (A) de la tête de col fémoral (4) et le point milieu (C) de l'axe épicondylien de l'os fémoral (1).

8. Dispositif pour déterminer une propriété caractéristique d'une structure anatomique (1), en particulier d'un os, comportant un ordinateur qui est configuré pour mettre en oeuvre le procédé selon l'une quelconque des revendications 1 à 7, ainsi qu'un dispositif de balayage pour déterminer des points de balayage sur la surface de la structure anatomique (1) afin de générer les données de balayage et/ou les données de points de repère qui sont transmises à l'ordinateur en vue d'un traitement.

9. Programme informatique qui, lorsqu'il est exécuté ou chargé sur un ordinateur amène l'ordinateur à mettre en oeuvre les étapes de procédé d'un procédé selon l'une quelconque des revendications 1 à 7.
